Europäisches Patentamt

European Patent Office

Office européen des brevets

(11) Veröffentlichungsnummer: **0 437 173 A1**

# (12) EUROPÄISCHE PATENTANMELDUNG

(21) Anmeldenummer: **90810129.8**

(22) Anmeldetag: **21.02.90**

(51) Int. Cl.5: **A61F 2/38**

(30) Priorität: **12.01.90 CH 92/90**

(43) Veröffentlichungstag der Anmeldung:
**17.07.91 Patentblatt 91/29**

(84) Benannte Vertragsstaaten:
**AT CH DE FR GB IT LI**

(71) Anmelder: **GEBRÜDER SULZER
AKTIENGESELLSCHAFT
Zürcherstrasse 9
CH-8401 Winterthur(CH)**

(72) Erfinder: **Schwägerl, Wolfgang, Prof. Dr.-med.
Dorotheergasse 9
A-1010 Wien(AT)**
Erfinder: **Oehy, Jürg
Büelhofstrasse 21
CH-8405 Winterthur(CH)**
Erfinder: **Koch, Rudolf
Oberdorfstrasse 229
CH-8267 Berlingen(CH)**

(54) **Als Führungselement für die Patella dienendes Implantat.**

(57) Das die Patella tragende Implantat besteht aus einem metallenen Trägerkörper (1) und einer Scheibe (8) als Führungselement für die Patella auf der künstlichen facies patellaris einer Kniegelenk-Prothese. Trägerkörper (1) und Scheibe (8) sind relativ zueinander drehbar miteinander verbunden.

Das neue Implantat ermöglicht es, dass beim Beugen und Strecken des Knies einerseits die Patella der "Zugrichtung" der an ihr angreifenden Muskel folgt und andererseits das Implantat mit seiner wulstartigen Erhebung (10) in der "Talsohle" der facies patellaris einer Kniegelenk-Prothese gleitet. Auf diese Weise werden unzulässige Reibung des Implantats auf dem Femurteil der Gelenkprothese und unnatürliche Belastungen des Muskel- und Bänderapparates vermieden.

Fig.1

## ALS FÜHRUNGSELEMENT FÜR DIE PATELLA DIENENDES IMPLANTAT

Die Erfindung betrifft ein als Führungselement für die Patella dienendes Implantat, welches auf einer Seite die Patella trägt und mit seiner anderen Seite, die als doppelt gekrümmte, sattelartige Fläche mit einer wulstartigen Erhebung längs eines Durchmessers ausgebildet ist, auf der facies patellaris zwischen den künstlichen Kondylen einer Kniegelenkprothese gleiten kann.

Bei physiologischer Bewegung des Kniegelenkes läuft die Patella bekanntlich nicht auf einer geometrisch ebenen Kurve. Vielmehr beschreibt die Patella infolge der Anatomie der Femurkondylen und der von den Muskeln ausgeübten Kräfte eine mehrfach gekrümmte Kurve. Bekannte Implantate der vorstehend genannten Art, die mit der Patella starr verbunden sind, stellen sich dabei relativ zur im allgemeinen als einfach gekrümmte, ebene Kurve ausgebildeten "Talsohle" der facies patellaris einer Kniegelenkprothese "quer". Dies hat zur Folge, dass sich die wulstartige Erhebung des Implantates auf den Femurteil der Kniegelenk-Prothese nur noch in einer punktförmigen Auflage abstützt, was zu einem unnatürlichen, aufgezwungenen Bewegungsablauf führt. Infolge des Muskelzuges resultieren daraus erhöhte Reibung mit Verschleiss und mitunter auch eine schmerzhafte Beeinträchtigung des Band-Apparates.

Aufgabe der Erfindung ist es, ein Implantat der eingangs genannten Art zu schaffen, durch das beim Beugen und Strecken des Kniegelenkes die natürliche Patella-Bewegung besser nachgeahmt wird. Erfindungsgemäss wird diese Aufgabe dadurch gelöst, dass das Implantat aus einem schalenartigen Trägerkörper für die Patella und einer die wulstartige Erhebung aufweisenden Scheibe besteht, die in der Schale des Trägerkörpers drehbar gelagert ist.

Durch die Trennung des Implantates in einen Träger für die Patella und eine dazu drehbare Scheibe als Führungselement in der facies patellaris können die Patella den Zug der an ihr angreifenden Muskeln in der "natürlichen Richtung" folgen und gleichzeitig die wulstartige Erhebung in einer Linienberührung in der künstlichen "Talsohle" der facies patellaris einer Kniegelenkprothese gleiten. Die relative Beweglichkeit zwischen Scheibe und Trägerkörper bewirkt gleichzeitig eine erhebliche Verringerung der auf die Verankerung zwischen Implantat und Patella wirkenden Belastungen.

Die neue Konstruktion hat darüberhinaus den Vorteil, dass - wegen der "Verstellbarkeit" des Winkels zwischen der Längsrichtung der wulstartigen Erhebung und der Zugrichtung der Muskel - die Arbeit des Operateurs erheblich erleichtert wird, da er bei der Implantation das Implantat nicht so exakt ausrichten muss, dass dieser Winkel "stimmt".

Als Verbindung zwischen Trägerkörper und Scheibe hat sich bewährt, wenn die Scheibe mit einem zapfenartigen Ansatz eine Hinterschneidung im Boden der Schale hintergreift, wobei der zapfenartige Ansatz mit vorspringenden Nasen in eine Ringnut der Hinterschneidung eingreift.

Ferner hat es sich als vorteilhaft erwiesen, wenn die ein Gleitelement bildende Scheibe aus einem Kunststoff, vorzugsweise Polyethylen, und der Trägerkörper aus Metall, vorzugsweise aus Titan oder einer Titanlegierung, gefertigt sind.

Im folgenden wird die Erfindung anhand eines Ausführungsbeispiels im Zusammenhang mit der Zeichnung näher erläutert.

Fig. 1    zeigt einen zur wulstartigen Erhebung senkrechten Schnitt I-I von Fig. 2 durch das neue Implantat;

Fig. 2    ist eine Aufsicht von oben auf den Trägerkörper nach Fig. 1;

Fig. 3, 4    geben in einer Ansicht von Fig. 1 von unten als Detail ein Beispiel für den Verschlussmechanismus der Verbindung zwischen Scheibe und Trägerkörper während des Einsetzens (Fig. 3) und in verriegeltem Zustand (Fig. 4) wieder.

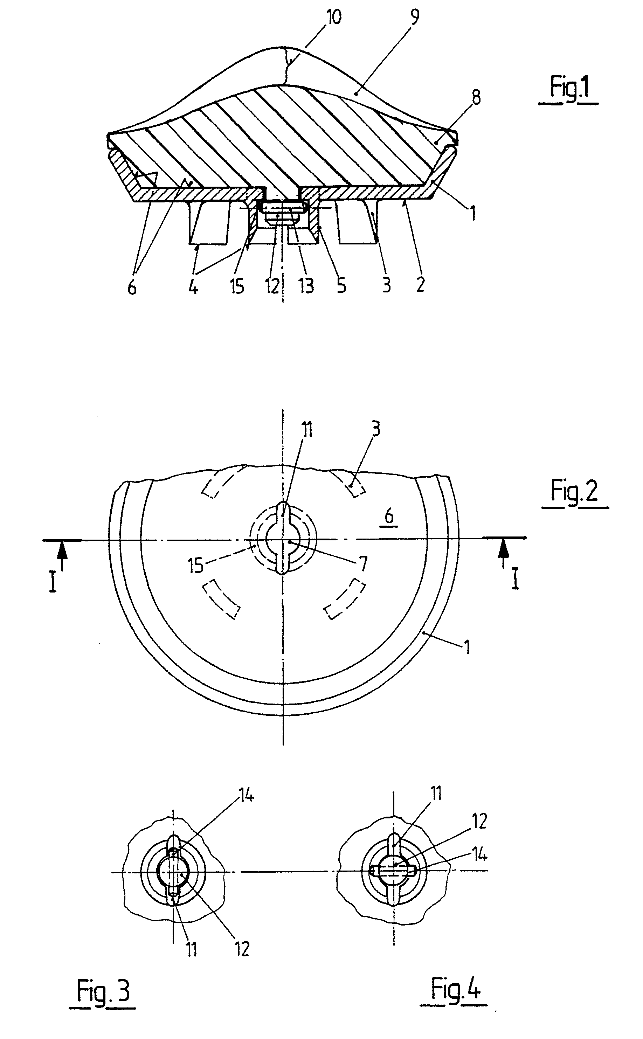

Ein schalenartiger, metallener Trägerkörper 1 (Fig. 1) ist an seinem Boden 2 mit auf seinem Umfang gleichmässig verteilten Flügeln 3 versehen, die in Schneidkanten 4 enden. Zusammen mit einem im Zentrum des Trägerkörpers 3 angeordneten Ring 5, dessen freies Ende ebenfalls eine Schneidkante 4 bildet, dienen die Flügel 3 zur Verankerung einer nicht dargestellten Patella auf dem Trägerkörper 1.

In die trogartige Schale 6 des Trägerkörpers 1 ist eine Kunststoff-Scheibe 8 eingelegt, deren freien Oberfläche als doppelt gekrümmte sattelartige Fläche 9 ausgebildet ist. Diese Fläche 9 bildet längs eines Durchmessers der Scheibe 8 eine wulstartige Erhebung 10, die als Führungselement für das Implantat in der "Talsohle" der facies patellaris eines nicht gezeigten Femurteils einer Kniegelenk-Prothese gleiten kann.

Der Ring 5, der mit der Schale 6 des Trägerkörpers 1 über einen, längs eines Durchmessers durch einen Schlitz 11 (Fig. 2) erweiterte Bohrung 7 in Verbindung steht, schirmt einen Verschlussmechanismus, durch den der Trägerkörper 1 mit der Kunststoffscheibe 8 verbunden ist, gegen das Einwachsen von Gewebe ab. Diese Verbindung besteht im vorliegenden Beispiels aus einem zapfenartigen Ansatz 12 an der Unterseite der Kunststoffscheibe 8. In dem Zapfen 12 ist eine horizontale Bohrung 13 vorgesehen, in der ein Haltestift 14 gelagert ist.

Wie in Fig. 3 gezeigt, wird die Scheibe 8 mit ihrem Zapfen 12 bei der Montage zunächst so in die Bohrung 7 eingeführt, dass der Stift 14 parallel zum Schlitz 11 verläuft. Anschliessend wird die Scheibe 8 um etwa 90° gedreht, so dass der Stift 14, wie in Fig. 4 gezeigt, eine ringförmige Hinterschneidung 15 innerhalb des Ringes 5 hintergreift. Die Scheibe 8 wird auf diese Weise mit dem Trägerkörper 1 verriegelt. Einerseits ist die Scheibe 8 dadurch drehbar gegenüber dem Trägerkörper 1, andererseits haben die Drehungen der beiden Implantat-Teile relativ zueinander so geringe Amplituden, dass der geschilderte Verschluss Gewähr für eine ausreichende Verbindung beider Teile bietet.

## Patentansprüche

1. Als Führungselement für die Patella dienendes Implantat, welches auf einer Seite die Patella trägt und mit seiner anderen Seite, die als doppelt gekrümmte, sattelartige Fläche (9) mit einer wulstartigen Erhebung (10) längs eines Durchmessers ausgebildet ist, auf der facies patellaris zwischen den künstlichen Kondylen einer Kniegelenkprothese gleiten kann, dadurch gekennzeichnet, dass das Implantat aus einem schalenartigen Trägerkörper (1) für die Patella und einer die wulstartige Erhebung (10) aufweisenden Scheibe (8) besteht, die in der Schale (6) des Trägerkörpers (1) drehbar gelagert ist.

2. Implantat nach Anspruch 1, dadurch gekennzeichnet, dass die Scheibe (8) mit einem zapfenartigen Ansatz (12) eine Hinterschneidung (15) im Boden der Schale (6) hintergreift.

3. Implantat nach Anspruch 2, dadurch gekennzeichnet, dass der zapfenartige Ansatz (12) mit vorspringenden Nasen (14) in eine Ringnut der Hinterschneidung (15) eingreift.

4. Implantat nach einem der Ansprüche 1 bis 3, dadurch gekennzeichnet, dass der Trägerkörper (1) aus Metall, vorzugsweise aus Titan oder einer Titan-Legierung besteht, während die Scheibe (8) aus Kunststoff, vorzugsweise aus Polyethylen, gefertigt ist.

Fig.1

Fig.2

Fig.3

Fig.4

**Europäisches Patentamt**

**EUROPÄISCHER RECHERCHENBERICHT**

Nummer der Anmeldung

**EP 90 81 0129**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-0 021 421 (BIOMEDICAL ENGINEERING)<br>* Seite 29, Zeile 1 - Seite 31, Zeile 6; Figuren 10,12-14,26 *<br>– – – | 1-4 | A 61 F<br>2/38 |
| X | DE-U-8 325 767 (M. IZADPANAH)<br>* Ansprüche 1,3-5; Figuren 6-8 *<br>– – – | 1-4 | |
| A | FR-A-2 594 323 (MATCO)<br>* Seite 3, Zeile 30 - Seite 4, Zeile 23; Figuren 1,5 *<br>– – – | 1-2 | |
| A | US-A-4 094 017 (L.S. MATTHEWS et al.)<br>* Spalte 5, Zeilen 26-45; Figuren 11-12 *<br>– – – – – | 1 | |
| | | | **RECHERCHIERTE SACHGEBIETE (Int. Cl.5)**<br><br>A 61 F |

Der vorliegende Recherchenbericht wurde für alle Patentansprüche erstellt

| Recherchenort | Abschlußdatum der Recherche | Prüfer |
|---|---|---|
| Den Haag | 08 März 91 | NICE P.R. |